# EUROPEAN PATENT SPECIFICATION

(11) **EP 4 035 612 B1**
(45) Date of publication and mention of the grant of the patent: **01.11.2023**
(21) Application number: 20803625.1
(22) Date of filing: 25.09.2020
(51) Int. Cl.: A61B 17/16, A61B 17/88, A61F 2/46

(54) **CHISEL FOR PLACING AN ANCHOR IN A VERTEBRA**
MEISSEL ZUM SETZEN EINES ANKERS IN EINEM WIRBEL
CISEAU POUR PLACER UN ANCRAGE DANS UNE VERTÈBRE

(30) Priority: 27.09.2019 ES 201931564 U
(43) Date of publication of application: 03.08.2022
(73) Proprietor: Neos Surgery, S.L., 08290 Cerdanyola del Vallès (ES)
(72) Inventor: SERRAHIMA TORNEL, Marc, 08195 SANT CUGAT DEL VALLÈS (ES); LLAS VARGAS, Salvador, 08290 CERDANYOLA DEL VALLÈS (ES); RODRIGUEZ ALONSO, Ana, 08290 CERDANYOLA DEL VALLÈS (ES); CHICO ROCA, Lluis, 08911 BADALONA (ES)
(74) Representative: Sugrañes, S.L.P.
(86) International application number: PCT/ES2020/070574
(87) International publication number: WO 2021/058848

(56) References cited:
- WO-A2-2009/094493
- US-A1- 2017 007 409

## Description

### Technical field of the invention

The chisel of the present invention is specially adapted for placing an anchor in a vertebra in order to subsequently be able to couple a prosthesis to the anchor, such that the prosthesis is properly positioned in the vertebra.

### Background of the invention

During a surgical intervention to place a vertebral prosthesis, the positioning of the prosthesis to be placed in the vertebra must be carried out with precision, since an incorrect placement could prevent the correct functioning of the prosthesis, it even being necessary to intervene surgically again to reposition or remove the prosthesis.

For the placement of prostheses in vertebrae, solutions are known based on first incorporating an anchor in a vertebra and subsequently coupling a prosthesis to said anchor, thus allowing, if the anchor is properly placed in the vertebra, the prosthesis to be properly placed when coupling the prosthesis to the anchor.

This type of anchor can be placed by means of a chisel that enables the vertebra to be carved to expose the area in which the anchor will be fixed, to subsequently place the anchor in the exposed area carved by means of the chisel, for example, by screwing it with a screwdriver.

However, as these types of anchors are very small, during the surgical intervention it is extremely difficult to be able to ensure that the anchor is properly placed in the vertebra, which can lead to unnecessary wear of the vertebra if several screwing attempts are carried out. Furthermore, it is also very difficult to insert the anchor sufficiently for it to be correctly secured to the vertebra without it being excessively inserted and not enabling the prosthesis to be correctly coupled.

It is therefore an objective of the present invention to disclose a chisel that enables an anchor to be correctly placed in a vertebra so that a prosthesis subsequently coupled to the anchor is properly positioned.

It is also another objective of the present invention to disclose a kit that enables a prosthesis to be placed in a vertebra using this chisel.

Another objective is to disclose an alternative to known systems for placing vertebral prostheses.

Document US2017/007409 A1 discloses an exemplary chisel comprising a cutting mouth adapted for carving a vertebra with a hammer blow in a carving direction, and a handle provided with a fixed handle portion, the cutting mouth and the fixed handle portion being joined by an arm provided with a conduit with an outlet.

### Explanation of the invention

The chisel for placing an anchor in a vertebra of the present invention is one of those comprising a cutting mouth adapted for carving a vertebra with a hammer blow in a carving direction, and a handle, adapted for receiving the hammer blows and transmitting them to the cutting mouth. Essentially, the chisel is characterised in that the handle is provided with a fixed handle portion and a rotatable handle portion with respect to the cutting mouth, the cutting mouth and the fixed handle portion being joined by an arm provided with a conduit with an outlet that determines an outlet direction of the conduit, the rotatable handle portion being provided with a rod with a flexible portion and screwing means at the end thereof adapted for screwing an anchor, the rod being adapted for being inserted into the conduit and for advancing in the conduit by rotating the rotatable handle portion with respect to the cutting mouth to screw and place an anchor in a vertebra previously carved in the outlet direction of the conduit, such that the anchor is properly positioned in the vertebra for subsequently coupling a prosthesis.

Preferably, the cutting mouth comprises a punch and a cutting fin, thus enabling the punch to mark and start perforating a point in the vertebra during the carving operation of a vertebra, from which the cutting fin will carve the vertebra, the vertebra portion cut by the fin further enabling the passage of a joining appendix of the prosthesis.

In one embodiment, the cutting fin comprises a pointed projection that helps aid the initial positioning of the chisel against the vertebra, leaving the point slightly embedded in the vertebra, preventing the chisel from moving before carving.

In an alternative embodiment, the cutting fin comprises a cutting edge between the punch and the pointed projection, enabling a cut to be made from the base of the vertebra to be carved, between the pointed projection and the punch. This cutting edge can have a general C-shape, thus enabling it to enter the vertebra progressively, facilitating the cutting thereof.

In a variant of interest, the end of the punch is advanced in the carving direction with respect to the end of the pointed projection, such that when the chisel is applied against the vertebra, the end of the pointed projection is slightly embedded in the vertebra when the punch has already cut a portion of the base of the vertebra and has entered the intervertebral space by piercing the annulus fibrosus arranged between the vertebrae.

In a variant embodiment, the cutting edge extends radially with respect to the carving direction, in order to thus enable a straight cut in the vertebra to be made that subsequently enables a portion of a prosthesis that is secured in the anchor to be housed. The cutting edge is intended to extend radially with respect to the carving direction in a vertical direction, to thus form a vertical straight cut in the vertebra.

The cutting mouth of the chisel may be provided with guiding means of the carving direction, such that tracking is possible during the carving operation of the vertebra in order to verify that the vertebra is being carved in the correct direction. These guiding means of the carving direction can be a set of perforations aligned in the cutting mouth, such that if the cutting mouth is metal, it will be possible to track the carving direction for example by means of X-ray image. Naturally, the same effect will be achieved when the cutting mouth is radio-opaque to the rays from which the tracking image is generated.

It is disclosed that the cutting mouth can also be provided with a maximum carving mark, which can also be a perforation in the cutting mouth, such that if the cutting mouth is metal, it will be possible to track the carving depth, for example, by means of X-ray image.

In a variant embodiment, the fixed handle portion is provided with a threaded shaft, and the rotatable handle portion is provided with a thread complementary to the threaded shaft and adapted for enabling the rotatable handle portion to rotate with respect to the fixed handle portion, thus enabling the speed at which the rod advances along the conduit when rotating the rotatable handle portion to be adjusted, appropriately sizing the passage of this threaded shaft and thread. Naturally, it is intended that the thread passage of the handle is compatible with the thread passage of the anchor, such that the rotation and advancement of the rod can be correctly transmitted to the anchor during screwing of the anchor to the vertebra, for example, the thread passage of the anchor and that of the handle being equal. Alternatively, if the fixed handle portion is devoid of a threaded shaft, and the rotatable handle portion is devoid of a complementary thread, the advancement of the rod along the conduit will be conditioned by the thread passage of the anchor, when screwed to a vertebra.

The handle will preferably be provided with path limiting means of the rotation of the rotatable handle portion with respect to the fixed handle portion, to thus limit the advancement of the rod through the conduit and limit the screwing depth of the anchor in the vertebra and furthermore, to be able to determine the final angular position of the screwing means and, consequently, of the anchor screwed to the vertebra. Being able to determine the final angular position of the anchor screwed into the vertebra is important in order to thus ensure that a prosthesis that is subsequently coupled to the anchor is properly positioned. Preferably, when the fixed handle portion is provided with a threaded shaft, and the rotatable handle portion is provided with a thread complementary to the threaded shaft and adapted for enabling the rotatable handle portion to rotate with respect to the fixed handle portion, the path limiting means can be a path end or a stop formed in the complementary thread or the threaded shaft, which will limit the rotation of the rotatable handle portion with respect to the fixed handle portion, said path limiting means being able to be incorporated during the manufacture of the threaded shaft or complementary thread.

In one embodiment, the conduit comprises an angled section between the carving direction and the outlet direction, carrying out a smooth transition between the carving direction and the outlet direction of the conduit.

In one embodiment, the cutting mouth comprises support means that enable the cutting mouth to be supported on a lower vertebra while the upper vertebra is being carved, thus enabling the stability of the chisel to be improved and making it easier to follow a correct carving direction, as well as enabling the operation of screwing and anchor placing to be carried out without the chisel moving.

It is intended that these support means exhibit fins, such as two fins, which extend in opposite directions in a direction perpendicular to the carving direction, thus determining a suitable lower support surface for supporting the chisel on the vertebra immediately below during the carving operation.

The present invention further discloses a kit for applying a prosthesis to a vertebra which comprises a chisel as described above adapted for placing an anchor in a vertebra and an applicator equipment adapted for coupling a prosthesis to the anchor previously placed in the vertebra by means of the chisel.

The kit may further comprise an anchor adapted for being applied to a vertebra by means of the chisel and a prosthesis adapted for being coupled to the anchor by means of the applicator equipment.

The prosthesis of the kit may be provided with a sleeve, the applicator equipment being provided with securing means of the prosthesis adapted for securing the sleeve of the prosthesis and push means of the prosthesis, adapted for pushing the prosthesis out of the sleeve to couple it to the anchor.

In one embodiment, the applicator equipment of the kit comprises a rotatable grip connected to the push means, the grip being adapted for carrying out a linear movement of the push means when rotated.

It is disclosed that the rotatable grip of the applicator equipment of the kit is also connected with the securing means, the grip being adapted for carrying out a linear movement in the opposite direction to the linear movement of the push means when rotated.

It is also disclosed that the rotatable grip of the applicator equipment of the kit is connected to the securing means and the push means by means of respective concentric spindle nut assemblies coupled to concentric tubes.

Preferably, the tubes of the applicator equipment of the kit are removably coupled to the spindle nut assemblies of the applicator equipment, such that they can be separated from the spindle nut assemblies to be disinfected after each use of the kit.

### Brief description of the drawings

As a complement to the present description, and for the purpose of helping to make the features of the invention more readily understandable, said description is accompanied by a set of drawings which, by way of illustration and not limitation, represent the following:
Figure 1 shows a chisel according to the present invention;
Figure 2 shows the chisel of Fig. 1 disassembled;
Figure 3 shows a detailed cross section of the cutting mouth of the chisel of Fig. 1;
Figs. 4a to 4c show a screwing sequence of an anchor to a vertebra by means of the chisel of Fig. 1;
Figure 5 shows a kit which comprises a chisel according to the present invention:
Figure 6 shows an applicator device of the kit of Fig. 5;
Figure 7 shows a detail of the end of the applicator device of Fig. 6 with a prosthesis included;
Figure 8 shows a detailed view of the mechanism of the grip of the applicator device of Fig. 6;
Figs. 9a and 9b show a placing sequence of a prosthesis by means of the applicator device of Fig. 6 in an anchor previously screwed to a vertebra;
Figure 10 shows the applicator device of Fig. 6 disassembled;
Figs. 11 and 12 show a detail of the cutting mouth of another chisel according to the present invention;
and Figs. 13a to 13b show a screwing sequence of an anchor to a vertebra by means of the chisel provided with the cutting mouth of Figs. 11 and 12.

### Detailed description of the drawings

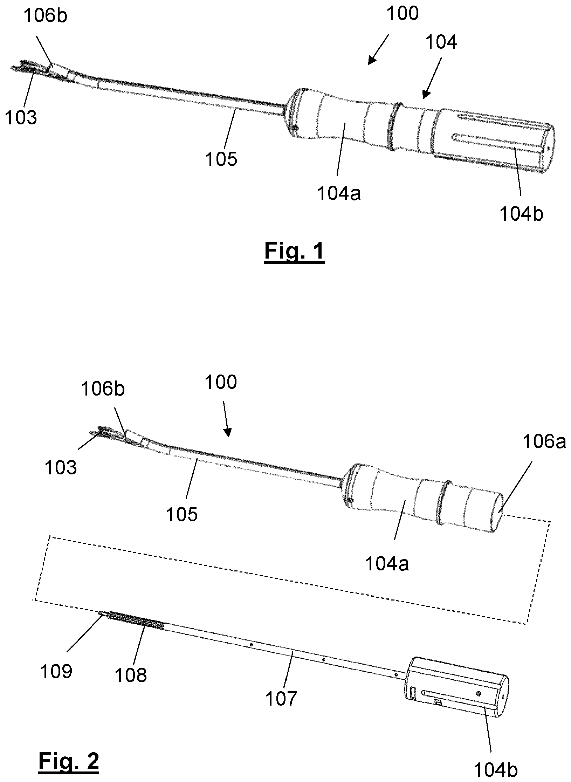

The chisel 100 shown in Fig. 1 is specially adapted for placing an anchor 200 in a vertebra. As can be seen, the chisel 100 comprises a cutting mouth 103 adapted for carving a vertebra with a hammer blow in a carving direction d1, and a handle 104, adapted for being struck by a hammer or the like, which is provided with a fixed handle portion 104a and a rotatable handle portion 104b with respect to the cutting mouth 103, the cutting mouth 103 and fixed handle portion 104a being joined by an arm 105. It should be noted that the carving direction d1 corresponds to the longitudinal direction of the chisel 100, such that the force exerted when striking the handle 104 is transmitted through the arm 105 to the cutting mouth 103, which will carve or cut a vertebra in that direction. The cutting mouth 103 is thus defined as the sharp part with which a chisel cuts, arranged at one end of the chisel.

As can also be seen in Fig. 2, the arm 105 that joins the cutting mouth 103 and the fixed handle portion 104a is advantageously provided with a conduit 106 with an outlet 106b in the arm 105 which determines an outlet direction d2 of the conduit 106 and an inlet 106a in the fixed handle portion 104a, the rotatable handle portion 104b being provided with a rod 107 with a flexible portion 108 and screwing means 109 at the end thereof, such as a screwdriver bit, adapted for securing and screwing an anchor 200, the rod 107 being adapted for being inserted into the conduit 106 and for advancing in the conduit 106 by rotating the rotatable handle portion 104b with respect to the cutting mouth 103 to screw and place an anchor 200 in the vertebra previously inserted in the conduit 106, by way of screwdriver. It is intended that the conduit 106 exhibits an angled section between the carving direction d1 and the outlet direction d2.

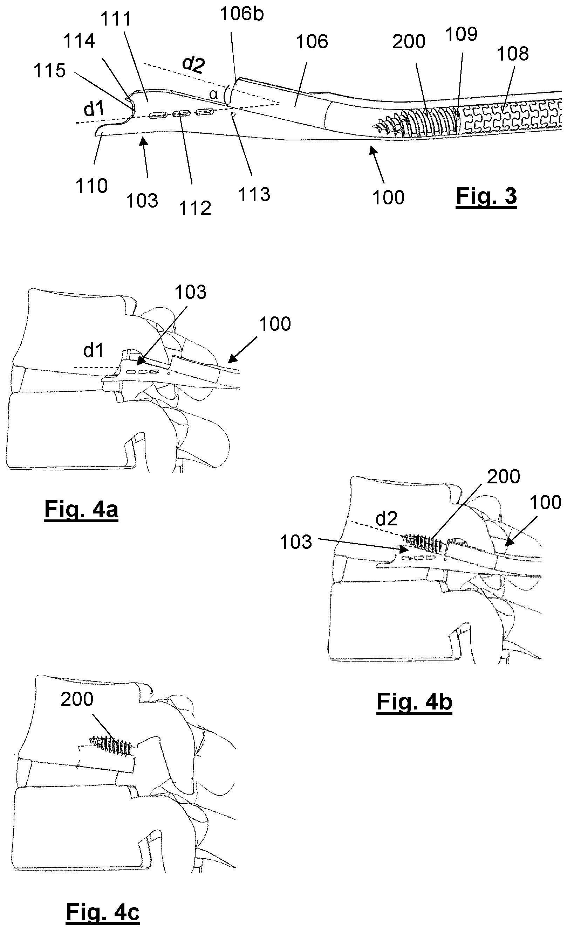

As can be seen in the detail of the end of the chisel 100 exhibited in Fig. 3, the cutting mouth 103 comprises a punch 110 and a cutting fin 111 arranged on the punch, such that the punch 110 enables the carving of the vertebra to start and thus being able to correctly direct the chisel 100 before the cutting fin 111 carves the vertebra to a greater extent, for example by opening a passageway to subsequently house a prosthesis appendage or arm in the anchor 200. It is further observed that the cutting fin 111 of the chisel 100 comprises a pointed projection 114 to favour the securing of the cutting fin 111 during the carving operation. For this securing to be achieved, the end of the punch 110 is advanced in the carving direction d1 with respect to the end of the pointed projection 114, such that when the pointed projection 114 is applied against the vertebra, the punch 110 has already entered the intervertebral space and carved the base of the vertebra. To carry out the carving operation, it is observed that the cutting fin 111 comprises a cutting edge 115 in a general C-shape that extends radially with respect to the carving direction d1, in order to thus enable a vertical straight cut to be formed in the vertebra between the punch 110 and the pointed projection 114.

Figs. 4a to 4c exhibit an exemplary placement sequence of an anchor 200 in a vertebra by means of the chisel 100 of the present invention, usually on the part of a surgeon during a prosthesis placement operation. Previously, an anchor 200 will have been placed in the chisel 100 in the conduit 106, preferably of circular cross section, secured by means of the screwing means 109 of the rod 107. First, the cutting mouth 103 of the chisel 100 will be placed against the vertebra onto which an anchor is to be screwed, as illustrated in Fig. 4a. After sufficiently carving the vertebra in the carving direction d1, as illustrated in Fig. 4a, the outlet direction d2 of the conduit 106 will advantageously enable the anchor 200 previously arranged in the conduit 106 and secured by means of the screwing means 109 of the rod 107 to be screwed in the outlet direction d2 as will be seen later in order to ensure the angle α with respect to the carving direction d1 in which the anchor 200 is fixed to a vertebra. Naturally, this angle α will be an angle that is preferably acute in order to make it easier for the anchor to enter the vertebra, and more preferably less than 45 degrees in order to favour that the anchor is arranged essentially perpendicular to the spinal column. This angle will be predetermined during the manufacture of the chisel 100, conveniently sizing and positioning the parts thereof.

In order to maintain the carving direction d1 and ensure that the chisel 100 is correctly inserted into the vertebra, the cutting mouth 103 comprises guiding means 112 of the carving direction d1, in this case a set of through holes aligned with respect to the carving direction d1 and which make it possible to track the carving of the chisel 100 by means of X-ray tracking or the like, such that the cutting mouth 103 being metal, the through holes are visible and the carving direction d1 can be tracked, enabling the surgeon to correct the carving direction d1.

Furthermore, it is intended that the cutting mouth 103 of the chisel 100 comprises a maximum carving mark 113, also by way of through hole, such that the surgeon can determine that the chisel 100 has already entered the vertebra sufficiently and that it is properly positioned to screw and place an anchor 200 previously placed in the conduit 106 in the vertebra.

At this time, when the chisel 100 is properly positioned, the surgeon must rotate the rotatable handle portion 104b, such that the screwing means 109 of the rod 107 advance rotating together with an anchor 200 placed in the screwing means 109 until exiting through the outlet 106b of the conduit 106, duly positioned and forming the predetermined angle α, such that, as the rotatable control portion 104b continues to rotate, the anchor 200 is screwed and inserted into the vertebra until it is sufficiently secured and placed in the vertebra, as illustrated in Fig. 4b, such that upon removal of the chisel 100, the anchor 200 is ready to subsequently receive a prosthesis, as shown in Fig. 4c. Furthermore, it should be noted that the cutting mouth 103 will have advantageously left a vertical straight cut in the vertebra suitable for subsequently housing an arm or appendix of the prosthesis. Naturally, depending on the type of prosthesis to be included in the anchor 200, the latter must be positioned at a greater or lesser angle with respect to the carving direction d1 and must be inserted to a greater or lesser extent, also depending on the dimensions thereof, which may vary, for example, depending on whether they must withstand a greater or lesser force.

Naturally, the fixed handle portion 104a may be provided, for example, with a threaded shaft, and the rotatable handle portion may be provided with a thread complementary to the threaded shaft and adapted for joining and enabling the rotatable handle portion 104b to rotate with respect to the fixed handle portion. In this case, by means of sizing the passage of this threaded shaft and thread, it is possible to adjust the speed at which the rod 107 advances along the conduit. It is also intended to be able to size the threaded shaft and thread, as well as the handle 104, such that only a movement of the rod 107 equal to the length that it is desired for the anchor 200 to be inserted into the vertebra is enabled. In this way, the fixed handle portion 104a and the rotatable handle portion 104b can be sized to act as a stop when the rotatable handle portion 104b has been sufficiently rotated. It is also observed that the rod 107 will preferably be hollow and will be provided with perforations that enable the interior thereof to be cleaned and disinfected after use thereof.

The handle 104 of the chisel 100 may also be provided with path limiting means of the rotation of the rotatable handle portion 104b with respect to the fixed handle portion 104a, for example, path limiting means of the rotation that not only enable the desired length for the anchor 200 to be inserted into the vertebra to be limited but also the final angular position thereof. These path limiting means of the rotation can be, for example, a path end or stop formed in the complementary thread or the threaded shaft. The final angular position in which the anchor 200 will be arranged in the vertebra can be important if it has components on the surface thereof, for example, a lateral channel into which an appendix or arm of the prosthesis must be subsequently inserted, which must be properly positioned.

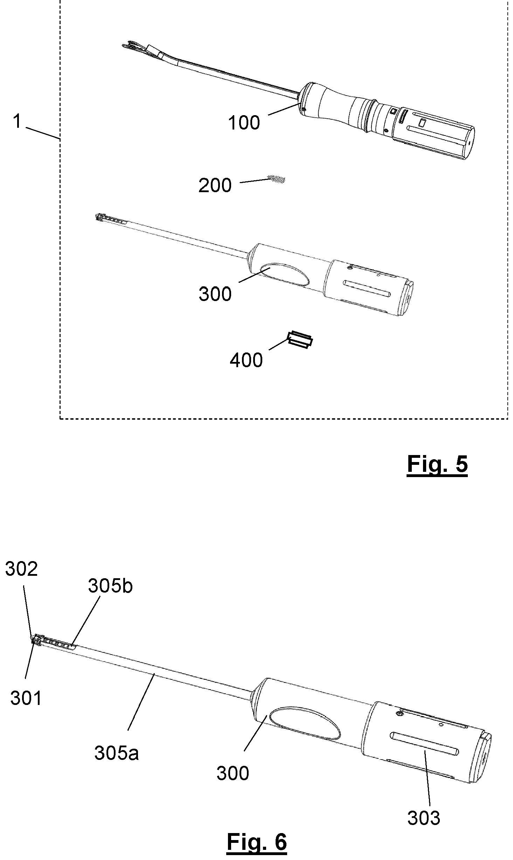

Figure 5 exhibits a kit 1 for applying a prosthesis 400 in a vertebra by means of an anchor 200 and which comprises a chisel 100 adapted for placing an anchor 200 in a vertebra as previously described and applicator equipment 300 adapted for coupling a prosthesis 400 to an anchor 200 previously placed in the vertebra. Naturally the kit 1 may further comprise an anchor 200 adapted for being applied to a vertebra by means of the chisel 100 described above and a prosthesis 400 adapted for being coupled to the anchor 200 by the applicator equipment 300. It is intended that the chisel 100, the anchor 200, the applicator equipment 300 and the prosthesis 400 that are part of the kit 1 can further be manufactured, marketed or exhibited separately and independently.

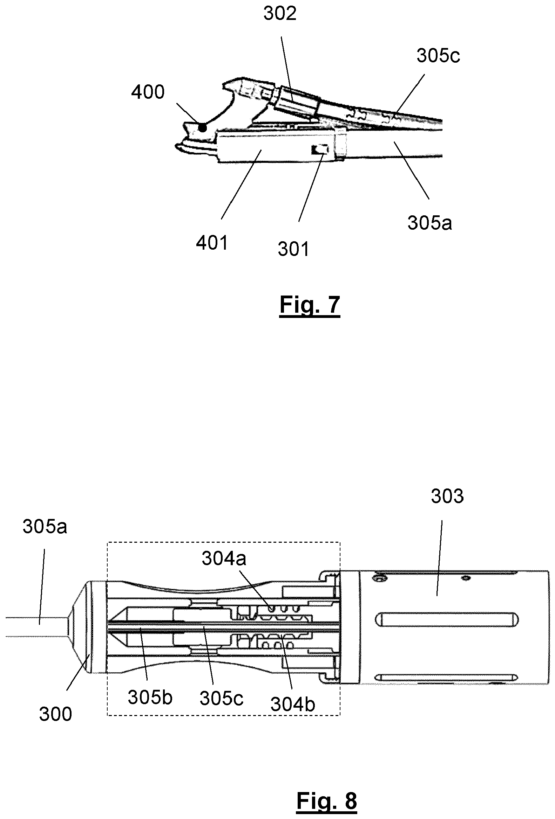

Figure 6 exhibits the applicator equipment 300 of the kit 1. As can be observed, the applicator equipment 300 comprises at the end thereof securing means 301 of the prosthesis 400, which are adapted for securing the sleeve 401 of the prosthesis 400, as shown in Fig. 7, and push means 302 of the prosthesis 400, adapted for pushing the prosthesis 400 out of the sleeve to couple it to the anchor 200.

In order to carry out the pushing action of the prosthesis 400, the applicator equipment 300 comprises a rotatable grip 303 connected to the push means 302 by means of a flexible screwdriver 305c, the grip 303 being adapted for carrying out a linear movement of the push means 302 when rotated and thus to progressively push the prosthesis 400 out of the sleeve to couple it to the anchor 200.

Furthermore, the rotatable grip 303 of the applicator equipment 300 is connected with the securing means 301, with the push means 302 and with auxiliary push means 306, by means of respective concentric spindle nut assemblies 304a, 304b coupled to respective concentric tubes 305a, 305b removably coupled to the respective spindle 304a, 304b thereof, as can be seen in Fig. 8, which illustrates a detailed cross section of the grip 303 of the applicator equipment 300.

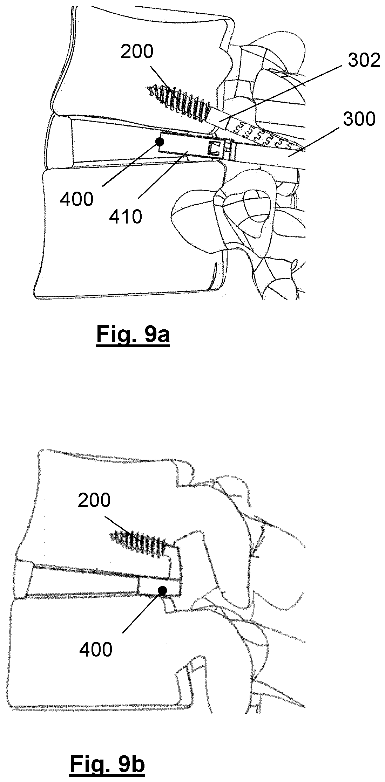

The applicator equipment 300 will enable a prosthesis 400 to be placed on an anchor 200 previously screwed into a vertebra, for example, in the manner previously described and illustrated in Fig. 4c. Thus, by means of the applicator equipment 300, the prosthesis 400 will be moved closer to the anchor 200 as indicated in Fig. 9a, then turning the grip 303 of the applicator equipment 300 to carry out the linear movement of the push means 302 and progressively push the prosthesis 400, assisted by auxiliary push means 306, out of the sleeve 401, to couple it to the anchor 200. The anchor 200 is intended to be provided with a rear entry through which a projection of the prosthesis 400 can be inserted and locked, for example, by means of a leaf spring mechanism arranged on the projection of the prosthesis 400. After the prosthesis 400 is correctly locked in the anchor 200, the applicator equipment 300 can be removed, leaving the prosthesis 400 arranged and secured to the vertebra in operative position as illustrated in Fig. 9b. Naturally, although a specific type of prosthesis 400 is exhibited to illustrate the invention, other different types of prosthesis could alternatively be placed in the anchor 200 screwed to the vertebra, as appropriate.

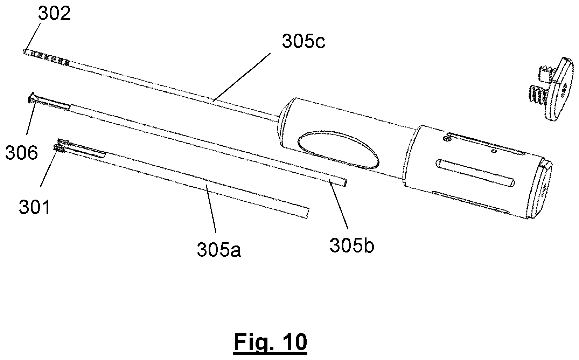

Advantageously, tubes 305a, 305b are intended to be removable from the respective spindle nut assemblies 304a, 304b thereof, as shown in Fig. 10, thus enabling the applicator equipment 300 to be washed and disinfected after each use.

It is also intended that the anchor 200 can be provided with a guide wire and the prosthesis 400 is provided with a through hole adapted for being traversed by this guide wire. In this case, the applicator equipment 300 could be provided with a passage adapted for receiving the end of the guide wire and thus easily guide the prosthesis 400 in the direction of the anchor 200. This guide wire can further be an electrical conductor, such that the applicator equipment 300 comprises an electrical warning circuit provided with two terminals adapted for generating a warning signal when the two terminals are electrically connected, one of the terminals being adapted for being connected to the guide wire and the other terminal being connected to the end of the push means 302.

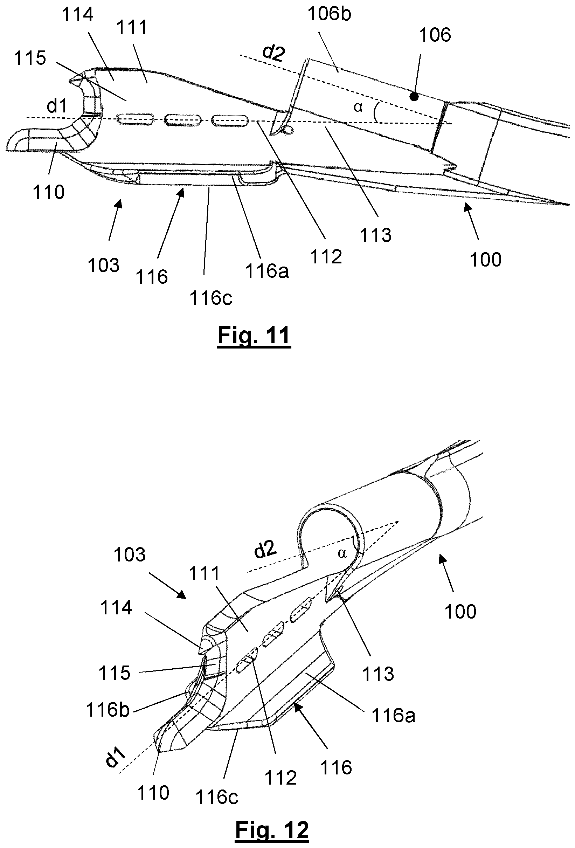

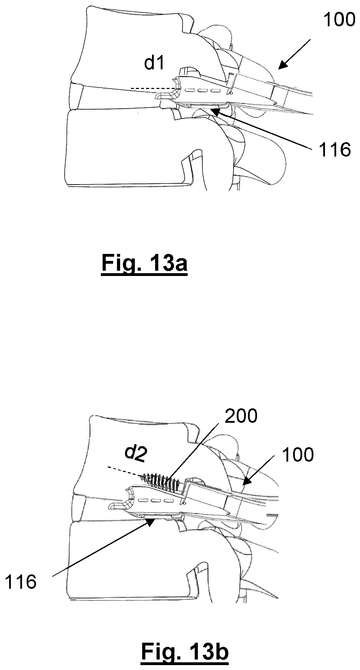

Figs. 11 and 12 exhibit another embodiment of the cutting mouth 103 of a chisel 100 according to the invention, the remainder of the chisel 100 being as described above. In this embodiment, the cutting mouth 103 comprises support means 116 to further facilitate the placing and carving of the chisel 100. These support means 116, which in the illustrated variant exhibit two fins 116a, 116b that extend in opposite directions in a direction perpendicular to the carving direction d1, determine a lower support surface 116c, such that the chisel 100 can be supported on a lower vertebra during the carving and screwing operation of the anchor as illustrated in Figs. 13a and 13b, the cutting edge 115 being inscribed in a plane perpendicular to the lower support surface 116c. Naturally, the thickness of the support means 116 will be suitably sized such that it occupies the intervertebral space, such that as the lower support surface 116c rests on a lower vertebra, the cutting mouth 103 is correctly arranged on the vertebra immediately above.

## Claims

1. A chisel (100) for placing an anchor (200) in a vertebra, which comprises a cutting mouth (103) adapted for carving a vertebra with a hammer blow in a carving direction (d1), and a handle (104) provided with a fixed handle portion (104a) and a rotatable handle portion (104b) with respect to the cutting mouth, the cutting mouth and the fixed handle portion being joined by an arm (105) provided with a conduit (106) with an outlet (106b) that determines an outlet direction of the conduit (d2), the rotatable handle portion being provided with a rod (107) with a flexible portion (108) and screwing means (109) at the end thereof adapted for screwing an anchor, the rod being adapted for being inserted into the conduit and for advancing in the conduit by rotating the rotatable handle portion with respect to the cutting mouth to screw and place an anchor in the vertebra previously carved in the outlet direction of the conduit.

2. The chisel (100) according to the preceding claim, **characterised in that** the cutting mouth (103) comprises a punch (110) and a cutting fin (111).

3. The chisel (100) according to the preceding claim, **characterised in that** the cutting fin (111) comprises a pointed projection (114).

4. The chisel (100) according to the preceding claim, **characterised in that** the cutting fin (111) comprises a cutting edge (115) between the punch (110) and the pointed projection (114).

5. The chisel (100) according to the preceding claim, **characterised in that** the cutting edge (115) extends radially with respect to the carving direction (d1).

6. The chisel (100) according to any one of claims 3 to 5, **characterised in that** the end of the punch (110) is advanced with respect to the end of the pointed projection (114) in the carving direction (d1).

7. The chisel (100) according to any one of the preceding claims, **characterised in that** the cutting mouth (103) comprises guiding means (112) of the carving direction (d1).

8. The chisel (100) according to any one of the preceding claims, **characterised in that** the cutting mouth (103) comprises a maximum carving mark (113).

9. The chisel (100) according to any one of the preceding claims, **characterised in that** the conduit (106) comprises an angled section.

10. The chisel (100) according to any one of the preceding claims, **characterised in that** the fixed handle portion (104a) is provided with a threaded shaft, and the rotatable handle portion (104b) is provided with a thread complementary to the threaded shaft and adapted for enabling the rotatable handle portion to rotate with respect to the fixed handle portion.

11. The chisel (100) according to any one of the preceding claims, **characterised in that** the handle (104) is provided with path limiting means of the rotation of the rotatable handle portion (104b) with respect to the fixed handle portion (104a).

12. The chisel (100) according to the preceding claim, **characterised in that** the fixed handle portion (104a) being provided with a threaded shaft, and the rotatable handle portion (104b) being provided with a thread complementary to the threaded shaft adapted for enabling the rotatable handle portion to rotate with respect to the fixed handle portion, the path limiting means are a path end or a stop formed in the complementary thread or the threaded shaft.

13. The chisel (100) according to any one of the preceding claims, **characterised in that** the cutting mouth (103) comprises support means (116).

14. The chisel (100) according to the preceding claim, **characterised in that** the support means (116) exhibit fins (116a, 116b) that extend in opposite directions in a direction perpendicular to the carving direction (d1), determining a lower support surface (116c).

15. A kit (1) for applying a prosthesis to a vertebra which comprises:
a. A chisel (100) according to any one of claims 1 to 14 adapted for placing an anchor (200) in a vertebra; and
b. An applicator equipment (300) adapted for coupling a prosthesis (400) in an anchor previously placed in the vertebra.

## Patentansprüche

1. Meißel (100) zum Setzen eines Ankers (200) in einem Wirbel, welcher einen schneidenden Mund (103), welcher dazu angepasst ist, einen Wirbel mit einem Hammerschlag in einer Schnitzrichtung (d1) zu schnitzen, und einen Griff (104), welcher mit einem feststehenden Griffteil (104a) und einem rotierbaren Griffteil (104b) in Bezug auf den schneidenden Mund versehen ist, umfasst, wobei der schneidende Mund und der feststehende Griffteil mittels eines Armes (105) verbunden ist, welcher mit einer Leitung (106) mit einem Austritt (106b) versehen ist, welcher eine Austrittsrichtung der Leitung (d2) bestimmt, wobei der rotierbare Griffteil mit einem Stab (107) mit einem flexiblen Teil (108) und Schraubmittel (109) am Ende desselben, welche dazu angepasst sind, einen Anker anzuschrauben, versehen ist, wobei der Stab dazu angepasst ist, in die Leitung eingesetzt zu werden und sich in der Leitung vorwärtszubewegen, indem der rotierbare Griffteil in Bezug auf den schneidenden Mund rotiert wird, um einen Anker im Wirbel, welcher zuvor geschnitzt wurde, in der Austrittsrichtung der Leitung anzuschrauben und zu platzieren.

2. Meißel (100) nach dem vorhergehenden Anspruch, **dadurch gekennzeichnet, dass** der schneidende Mund (103) einen Stempel (110) und eine Schneidrippe (111) umfasst.

3. Meißel (100) nach dem vorhergehenden Anspruch, **dadurch gekennzeichnet, dass** die Schneidrippe (111) einen spitzen Vorsprung (114) umfasst.

4. Meißel (100) nach dem vorhergehenden Anspruch, **dadurch gekennzeichnet, dass** die Schneidrippe (111) eine Schneidkante (115) zwischen dem Stempel (110) und dem spitzen Vorsprung (114) umfasst.

5. Meißel (100) nach dem vorhergehenden Anspruch, **dadurch gekennzeichnet, dass** sich die Schneidkante (115) radial in Bezug auf die Schnitzrichtung (d1) erstreckt.

6. Meißel (100) nach einem der Ansprüche 3 bis 5, **dadurch gekennzeichnet, dass** das Ende des Stempels (110) in Bezug auf das Ende des spitzen Vorsprungs (114) in die Schnitzrichtung (d1) vorgesetzt ist.

7. Meißel (100) nach einem der vorhergehenden Ansprüche, **dadurch gekennzeichnet, dass** der schneidende Mund (103) Führungsmittel (112) der Schnitzrichtung (d1) umfasst.

8. Meißel (100) nach einem der vorhergehenden Ansprüche, **dadurch gekennzeichnet, dass** der schneidende Mund (103) eine maximale Schnitzmarkierung (113) umfasst.

9. Meißel (100) nach einem der vorhergehenden Ansprüche, **dadurch gekennzeichnet, dass** die Leitung (106) einen abgewinkelten Bereich umfasst.

10. Meißel (100) nach einem der vorhergehenden Ansprüche, **dadurch gekennzeichnet, dass** der feststehende Griffteil (104a) mit einem Gewindeschaft versehen ist, und der rotierbare Griffteil (104b) mit einem zum Gewindeschaft komplementären Gewinde versehen ist und dazu angepasst ist, das Rotieren des rotierbaren Griffteils in Bezug auf den feststehenden Griffteil zu ermöglichen.

11. Meißel (100) nach einem der vorhergehenden Ansprüche, **dadurch gekennzeichnet, dass** der Griff (104) mit Wegbegrenzungsmittel der Rotation des rotierbaren Griffteils (104b) in Bezug auf den feststehenden Griffteil (104a) versehen ist.

12. Meißel (100) nach dem vorhergehenden Anspruch, **dadurch gekennzeichnet, dass** der feststehende Griffteil (104a) mit einem Gewindeschaft versehen ist, und der rotierbare Griffteil (104b) mit einem zum Gewindeschaft komplementären Gewinde versehen ist, welches dazu angepasst ist, die Rotation des rotierbaren Griffteils in Bezug auf den feststehenden Griffteil zu ermöglichen, wobei die Wegbegrenzungsmittel ein Wegende oder ein Anschlag, welcher im komplementären Gewinde oder im Gewindeschaft gebildet ist, sind.

13. Meißel (100) nach einem der vorhergehenden Ansprüche, **dadurch gekennzeichnet, dass** der schneidende Mund (103) Stützmittel (116) umfasst.

14. Meißel (100) nach dem vorhergehenden Anspruch, **dadurch gekennzeichnet, dass** die Stützmittel (116) Rippen (116a, 116b) aufweisen, welche sich in entgegengesetzten Richtungen in einer zur Schnitzrichtung (d1) senkrechten Richtung erstrecken, unter Bestimmung einer unteren Stützfläche (116c).

15. Kit (1) zum Aufbringen einer Prothese auf einem Wirbel, welches Folgendes umfasst:
a. einen Meißel (100) nach einem der Ansprüche 1 bis 14, welcher zum Setzen eines Ankers (200) in einem Wirbel angepasst ist; und
b. ein Applikatorgerät (300), welches dazu angepasst ist, eine Prothese (400) in einem im Wirbel zuvor platzierten Anker zu koppeln.

## Revendications

1. Ciseau (100) pour placer un ancrage (200) dans une vertèbre, qui comprend un mors tranchant (103) adapté pour sculpter une vertèbre avec un coup de marteau dans une direction de sculpture (d1), et un manche (104) pourvu d'une portion de manche fixe (104a) et d'une portion de manche rotative (104b) par rapport au mors tranchant, le mors tranchant et la portion de manche fixe étant jointes par un bras (105) pourvu d'un conduit (106) avec une sortie (106b) qui détermine une direction de sortie du conduit (d2), la portion de manche rotative étant pourvue d'une tige (107) avec une portion flexible (108) et des moyens de vissage (109) à l'extrémité de celle-ci adaptés pour visser un ancrage, la tige étant adaptée pour être insérée à l'intérieur du conduit et pour avancer dans le conduit par rotation de la portion de manche rotative par rapport au mors tranchant pour visser et placer un ancrage dans la vertèbre préalablement sculptée dans la direction de sortie du conduit.

2. Ciseau (100) selon la revendication précédente, **caractérisé en ce que** le mors tranchant (103) comprend un poinçon (110) et une ailette tranchante (111).

3. Ciseau (100) selon la revendication précédente, **caractérisé en ce que** l'ailette tranchante (111) comprend une saillie pointue (114).

4. Ciseau (100) selon la revendication précédente, **caractérisé en ce que** l'ailette tranchante (111) comprend un bord tranchant (115) entre le poinçon (110) et la saillie pointue (114).

5. Ciseau (100) selon la revendication précédente, **caractérisé en ce que** le bord tranchant (115) s'étend radialement par rapport à la direction de sculpture (d1).

6. Ciseau (100) selon l'une quelconque des revendications 3 à 5, **caractérisé en ce que** l'extrémité du poinçon (110) est avancée par rapport à l'extrémité de la saillie pointue (114) dans la direction de sculpture (d1).

7. Ciseau (100) selon l'une quelconque des revendications précédentes, **caractérisé en ce que** le mors tranchant (103) comprend des moyens de guidage (112) de la direction de direction de sculpture (d1).

8. Ciseau (100) selon l'une quelconque des revendications précédentes, **caractérisé en ce que** le mors tranchant (103) comprend une marque de sculpture maximale (113).

9. Ciseau (100) selon l'une quelconque des revendications précédentes, **caractérisé en ce que** le conduit (106) comprend une section inclinée.

10. Ciseau (100) selon l'une quelconque des revendications précédentes, **caractérisé en ce que** la portion de manche fixe (104a) est pourvue d'un arbre fileté et que la portion de manche rotative (104b) est pourvue d'un filetage complémentaire à l'arbre fileté et adapté pour permettre à la portion de manche rotative de tourner par rapport à la portion de manche fixe.

11. Ciseau (100) selon l'une quelconque des revendications précédentes, **caractérisé en ce que** le manche (104) est pourvu de moyens limiteurs de parcours de la rotation de la portion de manche rotative (104b) par rapport à la portion de manche fixe (104a).

12. Ciseau (100) selon la revendication précédente, **caractérisé en ce que**, la portion de manche fixe (104a) étant pourvue d'un arbre fileté, et la portion de manche rotative (104b) étant pourvue d'un filetage complémentaire à l'arbre fileté adapté pour permettre à la portion de manche rotative de tourner par rapport à la portion de manche fixe, les moyens limiteurs du parcours sont une extrémité de parcours ou une butée formée dans le filetage complémentaire ou l'arbre fileté.

13. Ciseau (100) selon l'une quelconque des revendications précédentes, **caractérisé en ce que** le mors tranchant (103) comprend des moyens de support (116).

14. Ciseau (100) selon la revendication précédente, **caractérisé en ce que** les moyens de support (116) présentent des ailettes (116a, 116b) qui s'étendent dans des directions opposées dans une direction perpendiculaire à la direction de sculpture (d1), déterminant une surface de support inférieure (116c).

15. Trousse (1) pour placer une prothèse dans une vertèbre, qui comprend :
a. un ciseau (100) selon l'une quelconque des revendications 1 à 14, adapté pour placer un ancrage (200) dans une vertèbre ; et
b. un équipement applicateur (300) adapté pour accoupler une prothèse (400) dans un ancrage préalablement placé dans la vertèbre.
